Europäisches Patentamt

European Patent Office

Office européen des brevets

(19)

(11) Veröffentlichungsnummer : **0 346 477 B1**

## (12) EUROPÄISCHE PATENTSCHRIFT

(45) Veröffentlichungstag der Patentschrift :
**30.12.92 Patentblatt 92/53**

(51) Int. Cl.⁵ : **A61F 13/15**

(21) Anmeldenummer : **88907824.2**

(22) Anmeldetag : **05.09.88**

(86) Internationale Anmeldenummer :
**PCT/JP88/00888**

(87) Internationale Veröffentlichungsnummer :
**WO 89/02228 23.03.89 Gazette 89/07**

(54) **WEGWERFWINDEL.**

(30) Priorität : **07.09.87 JP 223780/87**

(43) Veröffentlichungstag der Anmeldung :
**20.12.89 Patentblatt 89/51**

(45) Bekanntmachung des Hinweises auf die
Patenterteilung :
**30.12.92 Patentblatt 92/53**

(84) Benannte Vertragsstaaten :
**DE FR IT SE**

(56) Entgegenhaltungen :
**EP-A- 0 219 326**
**EP-A- 0 243 013**
**EP-A- 0 251 332**
**JP-A- 5 865 002**
**JP-A- 5 887 301**
**JP-A-61 296 103**
**JP-U- 6 288 704**

(73) Patentinhaber : **UNI-CHARM COMPANY
LIMITED
182, Shimobun Kinsei-Machi
Kawanoe-Shi Ehime-ken (JP)**

(72) Erfinder : **SUZUKI, Migaku
19-2, Ueki
Kamakura-shi Kanagawa 247 (JP)**
Erfinder : **OCHI, Mitsuzo
4-172, Ohaza-Fujiwara Doi-cho
Uma-gun Ehime 799-07 (JP)**
Erfinder : **KUDO, Takeshi
3389-11, Kawanoe-cho
Kawanoe-shi Ehime 799-01 (JP)**

(74) Vertreter : **Sperling, Rüdiger, Dipl.-Ing. et al
Patentanwälte Dipl.Ing.S. Staeger
Dipl.Ing.Dipl.Wirtsch.Ing. R Sperling
Müllerstrasse 31
W-8000 München 5 (DE)**

## Beschreibung

Die vorliegende Erfindung betrifft eine Wegwerfwindel des Typs, bei dem keine Windelabdeckung erforderlich ist, genauer gesagt, solche Windeln, die in den Bereichen, in denen die Schenkel und/oder Hüften umschlossen werden, mit auslaufverhindernden Mitteln versehen sind, die elastische Klappen aufweisen.

Stand der Technik:

In der EP-A- 219 326 sind Windeln beschrieben, die mit auslaufverhindernden Mitteln versehen sind, die elastische Klappen aufweisen, welche jeweils um die Schenkel des Babies herumgelegt werden. Diese in dieser Schrift offenbarten, ein Auslaufen verhindernden Mittel weisen erste Klappen auf, die sich nach außen erstrecken und jeweils mit elastischen Teilen versehen sind, sowie zweite Klappen, in deren Seitenkante ein elastisches Teil eingearbeitet ist, während die gegenüberliegende Seitenkante mit einem Hauptkörper des Artikels verbunden ist. Eine dieser Seitenkanten ist nach innen eingedrückt und an den in Längsrichtung gegenüberliegenden Enden befestigt.

Jede der zweiten Klappen ist als eine Tasche ausgebildet, um jeglichen Fluß von Ausscheidungen in Querrichtung zu unterbinden, wenn diese Klappe unter Einwirkung der ihr zugeordneten elastischen Mittel aufgerichtet wird. Bei einer Windel mit einem derartigen Aufbau sind die Taschen um die Schenkel des Trägers herum offen, wobei die zweiten Klappen unter der zusammenziehenden Kraft der zugeordneten elastischen Teile gegen den Schrittbereich aufgerichtet werden (als Schrittbereich wird der Bereich zwischen den beiden Schenkeln definiert). Gleichzeitig werden die ersten Klappen an die Innenseiten des Artikel gefaltet und unter dem Einfluß der zugeordneten elastischen Teile gegen die zugeorndeten Schenkel gepreßt. In diesem Zustand sind jeweilige Faltenlinien der ersten Klappen deckungsgleich mit den Basislinien der zugeordneten zweiten Klappen, d.h. die Linien, entlang welchen die zweiten Klappen mit dem Hauptkörper der Windel verbunden sind, und die zweiten Klappen werden hierbei nicht gegen die zugeordneten Schenkel gepreßt.

Es sei betont, daß das Ausmaß, mit welchem diese Taschen geöffnet werden, abhängt von dem Winkel, mit dem die zweiten Klappen aufgerichtet sind. Wenn beispielsweise der Träger eine Haltung oder eine Stellung der Beine einnimmt, bei welcher die Schenkel weit auseinandergespreizt sind, so daß die zweiten Klappen gestreckt sind, oder wenn der Träger sich setzt, wobei die zweiten Klappen nach unten gedrückt werden, sind diese Taschen ungenügend geöffnet, so daß die gewünschte blockierende Wirkung dieser Taschen gegen einen Fluß der Exkremente, insbesondere lose Ausscheidungen und Urin nicht aufrechterhalten werden kann, und unerfreuliche Auslauferscheinungen auftreten können.

Der Erfindung liegt als Hauptaufgabe zugrunde, eine Windel der genannten Gattung bereitzustellen, bei der in jeder Situation eine vollkommene Abdictung durch die ein Auslaufen verhindernden Mittel erzielt wird.

Die Aufgabe wird erfindungsgemäß bei einer Wegwerfwindel mit einer flüssigkeitsdurchlässigen oberen, einer flüssigkeitsundurchlässigen unteren Lage, einem zwischen beiden Lagen eingelegten Saugkern und mit im Schnittbereich an einander gegenüberliegenden Seiten angeordneten, ein Auslaufen verhindernden Mitteln, die eine Tasche ausbilden und jeweils eine biegsame Klappe und ein oder mehrere elastische Teile aufweisen, wobei :

- die Klappe weist einen sich von der Windel nach oben erstreckenden Stützabschnitt, einen inneren, sich von dem Stützabschnitt nach innen erstreckenden inneren Zweigabschnitt und einen äußeren, sich von dem Stützabschnitt nach außen erstreckenden Zweigabschnitt auf;
- das eine oder die mehreren elastische(n) Teil(e) ist(sind) zumindest teilweise in den inneren und äußeren Zweigabschnitten eingearbeitet und bilden elastische Falten; und
- zumindest die jeweiligen Innenflächen des Stützabschnitts und des inneren Zweigabschnitts bilden die Tasche zur Aufnahme von Ausscheidungen.

Es wird bevorzugt, ein Paar derartiger, ein Auslaufen verhindernden Mittel zwar im Schrittbereich aber um jeden Schenkel des Trägers herum anzuordnen, und zwar entlang einander gegenüberliegender, sich in Längsrichtung erstreckender Seiten des absorbierenden Kerns, so daß die inneren Zweigabschnitte üblicherweise unter der Wirkung der elastischen Mittel gegen die zugeordneten Schenkel und nicht in Richtung zum Schrittbereich gepreßt werden, so daß die zumindest durch die jeweiligen inneren Flächen der inneren Zweigabschnitte und der zugeordneten Stützabschnitte geformten Taschen in ihrer entsprechend geöffneten Position unabhängig von der Haltung oder Stellung des Trägers offen gehalten werden. Dies hat zur Folge, daß die Taschen das Auslaufen von Ausscheidungen, insbesondere losen Exkrementen und Urin wirksam verhindern können. Gleichzeitig wirken die inneren Zweigabschnitte mit den äußeren Zweigabschnitten zusammen, um einen hohen Abdichteffekt bereitzustellen, der notwendig ist, um das Auslaufen der Exkremente zu verhindern; dies wird dadurch erreicht, daß die äußeren Zweigabschnitte, die neben den inneren Zweigabschnitten verlaufen, ebenfalls elastisch gegen die zugeordneten Schenkel gepreßt werden.

Falls es gewünscht wird, können diese ein Auslaufen verhinderten Mittel ebenfalls entlang der Hüft- oder Gürtellinie der Windel vorgesehen sein, um denselben hohen Abdichteffekt zu erzielen.

Kurze Beschreibung der Figuren:

Es zeigen:
Fig. 1 eine Draufsicht eines ersten Ausführungsbeispiels einer erfindungsgemäßen Windel mit teilweise weggebrochenen Bereichen;
Fig. 2 einen Schnitt entlang der Linie 2-2 in Fig. 1, wobei lediglich die erfindungsgemäßen, ein Auslaufen verhindernden Mittel dargestellt sind;
Fig. 3 u.4 jeweils Teilansichten von Schnitten zweier Ausführungsvarianten der in Fig. 2 dargestellten, ein Auslaufen verhindernden Mittel;
Fig. 5 eine teilweise perspektivische Ansicht entsprechend der Ausbildung gem. Fig. 2;
Fig. 6-10 jeweils Schnitte weiterer Ausführungsvarianten der ein Auslaufen verhindernden Mittel;
Fig. 11 eine Draufsicht auf eine abgewickelte erfindungsgemäße Windel mit teilweise weggebrochenen Bereichen und
Fig. 12 eine perspektivische Ansicht der Windel aus Fig. 11.

Bevorzugte Ausführungsbeispiele der Erdindung:

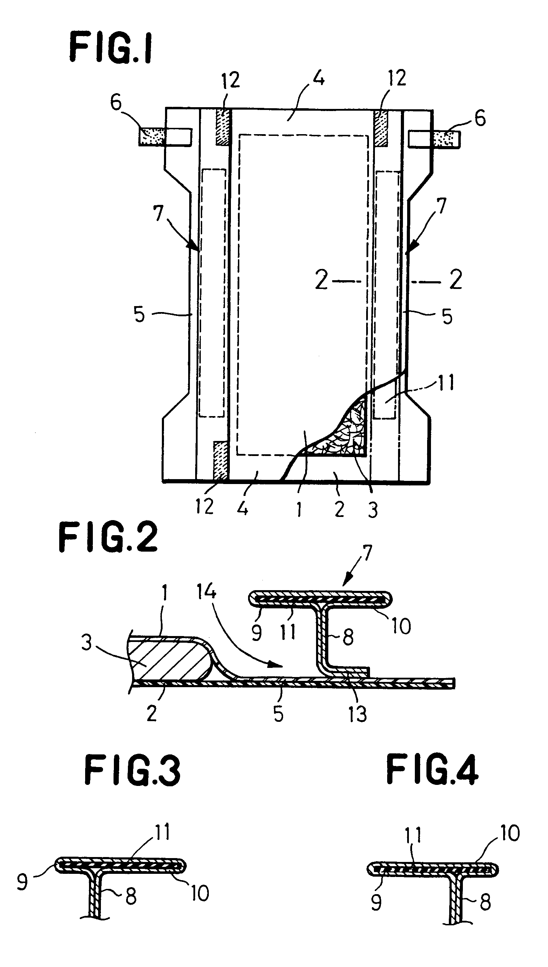

Im folgenden wird die Erfindung anhand der beigefügten Zeichnungen näher erläutert. Zunächst wird auf Fig. 1 Bezug genommen. Die darin dargestellte Wegwerfwindel weist eine flüssigkeitsdurchlässige obere Lage 1, eine flüssigkeitsundurchlässige untere Lage 2, einen zwischen diesen beiden Lagen eingelegten Saugkern 3, Hüft- und Seitenklappen 4,5, die jeweils von Abschnitten der beiden Lagen gebildet werden, die sich jeweils von den einander gegenüberliegenden Längs- bzw. Querrändern des Saugkerns nach außen erstrecken und Klebebänder 6 auf, die an den sich gegenüberliegenden seitlichen Enden der Hüftklappe befestigt sind.

Wie aus Fig. 2 weiter zu erkennen ist, weist die Wegwerfwindel noch zweite Klappen 7 auf, die sich längs der jeweiligen Klappen 5 erstrecken. Jede der Seitenklappen 7 ist aus einer einzigen Lage zu einer Hülse oder Schlaufe mit einem T-förmigen Querschnitt geformt mit einem Stützabschnitt 8, der oben auf der Oberfläche der zugeordneten Klappe 5 befestigt ist, einen inneren Zweigabschnitt 9, der sich von dem oberen Ende des Stützabschnitts nach innen erstreckt und einem äußeren Zweigabschnitt 10, der sich von dem oberen Ende des Stützabschnitts 8 nach außen erstreckt. Die inneren und äußeren Stützabschnitte 9,10 enthalten ein relativ breites elastisches Teil 11, das beiden Zweigabschnitten gemeinsam ist. Das elastische Teil 11 muß zumindest im Schrittbereich der Windel angeordnet sein.

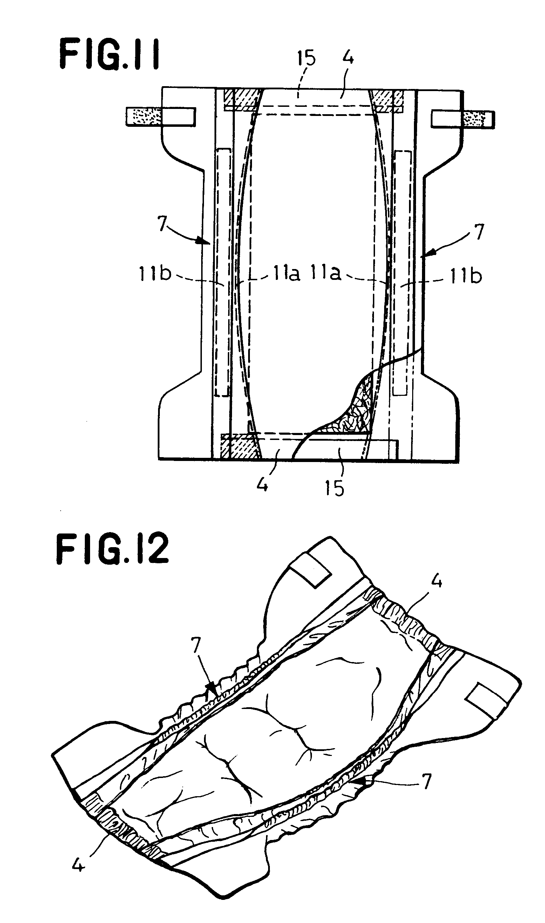

Der innere Zweigabschnitt 9 und/oder der äußere Zweigabschnitt 10 sind/ist vorzugsweise an der zugeordneten Klappe an den einander gegenüberliegenden Längsenden oder dem Längsende im Vorderbereich der Windel befestigt. Eine derartige Befestigung kann auf verschiedene Arten erzielt werden, z.B. durch Befestigung des inneren Zweigabschnitts 9 an den mit der Bezugsziffer 12 in Fig. 1 gekennzeichneten, einander gegenüberliegenden Längsenden, durch Befestigen des äußeren Zweigabschnitts 10 an den in Längsrichtung einander gegenüberliegenden Enden, durch Befestigen sowohl des inneren als auch des äußeren Zweigabschnitts 9,10 an den in Längsrichtung einander gegenüberliegenden Enden (vergl. Fig. 5), durch Befestigen des inneren Zweigabschnitts an dem Längsende, das im vorderen Bereich der Windel gelegen ist, durch Befestigen des äußeren Zweigabschnitts an dem Längsende, das im vorderen Bereich der Windel gelegen ist, oder durch Befestigen sowohl des inneren Zweigabschnitts als auch des äußeren Zweigabschnitts 9,10 an den jeweiligen Längsenden, die im vorderen Bereich der Windel vorgesehen sind.

Was die Breite anbetrifft, mit welcher sich die inneren und äußeren Zweigabschnitte 9,10 jeweils seitlich erstrecken, so kann die Breite des inneren Zweigabschnitts im wesentlichen gleich der Breite des äußeren Zweigabschnitts sein, so wie es in Fig.2 dargestellt ist. Der erstere kann jedoch auch schmäler sein als der letztere, wie in Fig. 3 dargestellt, oder der erstere kann auch breiter sein als der letztere, vergl. Fig. 4.

Durch die breite Ausbildung des inneren Zweigabschnitts 9 kommen die in der Tasche aufgefangenen Ausscheidungen nicht mit der Haut des Trägers in Berührung, wodurch ein besonders angenehmer Tragekomfort gegeben ist.

Die Art, wie der innere und der äußere Zweigabschnitt 9,10 an den sich gegenüberliegende Längsenden befestigt werden, wie auch das Maß der Breite, mit der sich diese Abschnitte seitlich erstrecken, wird so ausgewählt, daß optimale Tragebedingungen bei allgemeiner Berücksichtigung der verschiedenen Faktoren, wie Größe der Windel, Abstand des Saugkerns 3 zu jeder Klappe 7 und Höhe des Stützabschnitts 8 erzielt werden können.

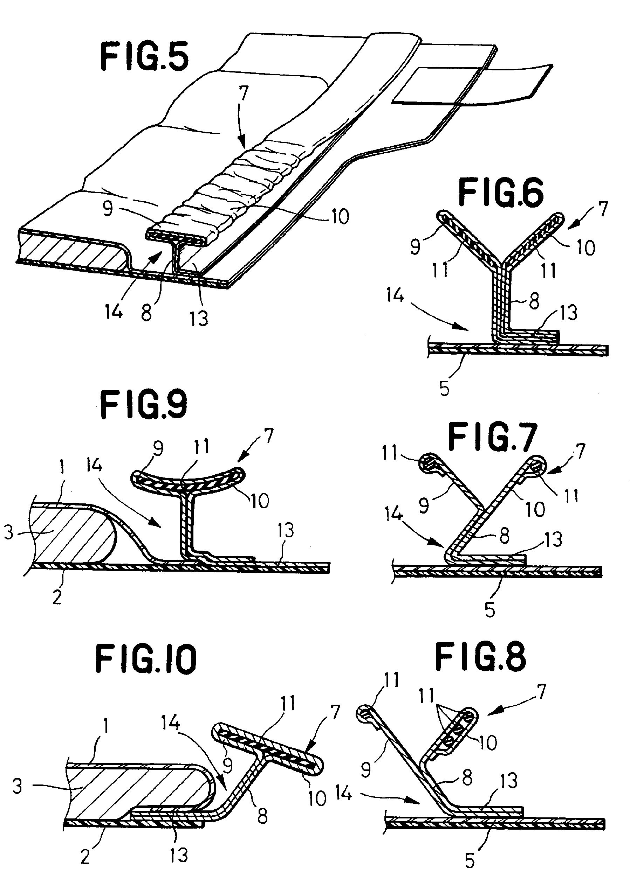

In der Figuren 6 bis 10 sind verschiedene Varianten des oben erläuterten Ausführungsbeispiels dargestellt, die die gleichen Wirkungen wie das beschriebene Ausführungsbeispiel haben.

In dem im Fig. 6 dargestellten Ausführungsbeispiel besteht jede Klappe 7 aus einem inneren und einem äußeren Abschnitt 9,10, die jeweils getrennt voneinander in entsprechende Schlaufen oder Hülsen geformt sind, die getrennte elastische Teile 11 umfassen.

Die Klappe 7 des in Fig. 7 dargestellten Ausführungsbeispiels weist einen inneren und einen äußeren Zweigabschnitt auf, deren jeweilige freie Außenränder ein zugeordnetes, schnurförmiges elastisches Teil 11 umhüllen.

Die in Fig. 8 dargestellten Klappe 7 weist einen inneren Zweigabschnitt 9 auf, dessen freie Randkante ein einziges faden- oder schnurförmiges elastisches Teil umhüllt, während der äußere Zweigabschnitt 10 im wesentlichen über seine gesamte Breite eine Vielzahl von fadenförmigen oder schnurförmigen elastischen Teilen 11 gemeinsam umschließt.

Die in Fig. 9 dargestellte Klappe 7 weist einen inneren und einen äußeren Zweigabschnitt 9 und 10 auf, die von einer einzigen Lage in eine T-förmige Hülse geformt sind. Die Abschnitte enthalten das elastische Teil 11, das einen konkav nach oben geöffneten Querschnitt aufweist. Bei diesem Ausführungsbeispiel ist die Breite der oberen Lage 1 geringer als die die unteren Lage 2, um die äußeren Seitenabschnitte der unteren Lage 2 frei von einer durchlässigen oberen Lage zu halten. An die Stelle dieser oberen Lage 1 tritt der Basisabschnitt 13 des Stützabschnitts 8, das auf die undurchlässige untere Lage 2 geklebt wird, um den zugeordneten äußeren Seitenbereich mit diesem Basisabschnitt 13 zu bedecken.

In den Ausführungsbeispielen gemäß der Figuren 6 bis 8 werden die Klappen 7 von zwei separaten Lagen gebildet, die entlang der beiden unteren Hälften aneinander geklebt sind, um den Stützabschnitt 8 zu bilden. Im oberen Bereich sind die beiden Lagen jeweils frei und zu den inneren bzw. äußeren Abschnitten 9 und 10 ausgebildet, wodurch sich eine Zweiglinie ergibt, die sich durch die gemeinsame Grenzlinie der Abschnitte 9 und 10 ergibt. Als Folge davon haben der innere und der äußere Abschnitt 9 und 10 jeweils die Tendenz, sich nach oben entlang der Zweiglinie zu einem V aufzubiegen, wenn die entsprechenden elastischen Teile 11 sich in einem gewissen Ausmaß zusammenziehen. Während die inneren und äußeren Zweigabschnitte 9,10 der Ausführungsbeispiele der Figuren 2 und 4 und 9 ebenfalls dazu tendieren, sich nach oben zu biegen, hier jedoch U-förmig und zwar abhängig von dem Ausmaß, mit welchem sich das elastische Teil 11 zusammenzieht, so ist dieses V-förmige Ausbilden der Ausführungsbeispiele der Figuren 6 bis 8 signifikanter als bei den Ausführungsbeispielen gemäß der Figuren 2 bis 4 und 9. Auch bei den Ausführungsbeispielen gemäß der Figuren 6 bis 9 kann die Breite, mit der sich der innere bzw. der äußere Abschnitt 9 bzw. 10 nach außen erstreckt, wie auch die Art und Weise, wie diese Abschnitte an den Längsenden oder einem Längsende festgelegt sind, in entsprechender Weise wie bei den Figuren 2 bis 4 ausgeführt werden.

In den in den Figuren 2 bis 9 dargestellten Ausführungsbeispielen erstreckt sind das Basisende 13 des Stützabschnitts 8 vorzugsweise bezüglich des Stützabschnitts nach außen, die vorliegende Erfindung ist jedoch nicht auf eine solche Anordnung beschränkt.

Die Klappe 7 des Ausführungsbeispiels der Fig. 10 ist in Form und Aufbau identisch zu dem Ausführungsbeispiel gemäß Fig. 2 mit der Ausnahme, daß im ersteren das Basisende 13 des Stützabschnitts 8 zwischen dem Abschnitt der oberen Lage 1, der unter den Seitenrand des Saugkerns 3 eingeschlagen ist und dem Abschnitt der unteren Lage 2, die den genannten Abschnitt der oberen Lage 1 abdeckt, zwischengelegt und daran befestigt.

In all den Ausführungen, die in den Figuren 2 bis 10 dargestellt sind, wird durch die Innenflächen des Stützabschnitts 8 und des inneren Zweigabschnitts 9 eine Tasche 14 gebildet. Während die Tasche gebildet wird, selbst wenn der innere Zweigabschnitt und/oder der äußere Zweigabschnitt 10 an den Längsenden festgelegt sind, wird es bevorzugt, eine derartige Festlegung so durchzuführen, daß die Richtung, in der die Tasche geöffnet ist, stabil ist.

Die Figuren 11 und 12 zeigen eine Variante des inneren Zweigabschnitts der vorgenannten Ausführungsformen. Gemäß dieser Variante folgt das Paar der einander gegenüberliegenden inneren Zweigabschnitte 9 jeweils den Kurven, die sich einander graduell nähern, um den mittleren Bereich der vorderen und rückwärtigen Bereiche der Wegwerfwindel zu bilden, wobei die schnur- oder fadenförmigen elastische Teile 11a entlang dieser jeweiligen Kurven angeordnet sind. Entlang der äußeren Zweigabschnitte 10 sind relativ breite elastische Teile 11b eingearbeitet. Bei dieser Ausführungsform sind relativ breite elastische Teile 15 zwischen der oberen Lage 1 und der rückwärtigen Lage 2 entlang der jeweiligen Hüftklappen 4 vorgesehen.

Die obere Lage 1 kann aus einem Faservlies, einem porösen Kunststoffilm oder ähnlichem hergestellt sein, die untere Lage kann aus einem luftdurchlässigen Kunststoffilm, einer Laminatlage eines solchen Films und einem Faservlies oder ähnlichem, der Saugkern 3 aus einer flockigen Pulpe, einer Mischung aus einer solchen Pulpe und hoch saugfähigen Polymerpartikeln oder einem ähnlichen Material bestehen. Daher können die verschiedenen Materialarten, die üblicherweise für eine derartige Windel verwendet werden, wahlweise benutzt werden, jedoch ist die vorliegende Erfindung nicht auf die Verwendung dieser Materialien beschränkt.

Die Klappe wird vorzugweise aus einem Material hergestellt, das zumindest teileweise so flexibel wie möglich ist, luftdurchlässig und wasserundurchlässig. Die Klappe 7 kann luftdurchlässig und wasserundurchlässig im Stützabschnitt 8 sein, am äußeren Zweigabschnitt 10, am Stützabschnitt 8 und dem äußeren Abschnitt 10, oder dem Stützabschnitt 8 und dem inneren Abschnitt 9 wie auch am äußeren Zweigabschnitt 10. Solches Material, das luftdurchlässig und wasserundurchlässig ist, umfaßt Faservliese, poröse Kunststoffilme und Laminate daraus.

Das elastische Teil 11 kann aus einem Material hergestellt sein, das üblicherweise für ein derartiges Teil verwendet wird, z.B. natürlicher oder synthetischer Gummi, Kunststoffe, wie Polyurethan oder andere Kunststoffe, die dazu neigen, Elastizität während einer Wärmebehandlung zu entwickeln oder beizubehalten. Die vorliegende Erfindung ist jedoch nicht auf die Verwendung eines solchen besonderen Material beschränkt. Es versteht sich, daß wenn ein relativ breites elastisches Teil verwendet wird, wie im Fall des Ausführungsbeispiels gem. der Figuren 2 bis 6, 19 und 11 (hier das Bezugszeichen 11b) Polyurethanschaum vorzugsweise verwendet werden kann.

Obwohl die Klappen 7, von denen jede einen Stützabschnitt 8 sowie einen inneren und einen äußeren Zweigabschnitt 9 und 10 aufweist, als im Schrittbereich der Wegwerfwindel angeordnet beschrieben worden sind, können die Klappen 7 auch in der rückwärtigen Hüftklappe 4 und den Klappen 5 im Schrittbereich oder nur in den Klappen 4 vorgesehen sein.

Das Ausbilden der Klappen 7 im Schrittbereich der Wegwerfwindel als Mittel zum Verhindern des Auslaufens um die jeweiligen Schenkel herum in der Weise, wie es oben beschrieben worden ist, stellt sicher, daß, wenn die Windel angelegt worden ist, der innere und der äußere Zweigabschnitt 9 und 10 elastisch gegen die jeweiligen Schenkel angepreßt werden, ohne in den Bereich, der zwischen den Schenklen definiert ist, zu verbleiben und daß die Taschen 14, die zumindest von den jeweiligen Innenflächen des Stützabschnitts 8 und den zugeordneten inneren Zweigabschnitten 9 gebildet werden, zuverlässig jedes Ausfließen von Auscheidungen verhindern. Wenn die HÜftklappe 4 der Wegwerfwindel mit der Klappe 7 als auslaufverhinderndes Mittel um die Hüfte herum vorgesehen ist, so ist sichergestellt, daß die inneren und äußeren Zweigabschnitte 9 und 10 elastisch gegen die Hüfte gepreßt werden und die Tasche 14, die zumindest von den jeweiligen Innenflächen des Stützabschnitts 8 und des inneren Zweigabschnitts 9 gebildet wird, zuverlässig jedes Ausfließen von Ausscheidungen verhindert. Es sei darauf hingewiesen, daß die Funktion und die Wirkung, die durch ein solches, ein Ausfließen verhinderndes Mittel bereitgestellt wird, optimal werden, wenn derartige Mittel an einander gegenüberliegenden Seiten der Windel im Schrittbereich ausgebildet sind.

Gewerbliches Anwendungsgebiet:

Aus der vorangegangenen Beschreibung ist es erkennbar, daß die erfindungsgemäß ausgebildete Wegwerfwindel geeignet ist als eine Windel, die keinerlei zusätzliche Windelabdeckung erfordert. Die in dieser Windel eingearbeiteten, ein Auslaufen verhindernden Mittel sind zusammen mit elastischen Haltemitteln nicht nur als Abdichtung um die Schenkel nützlich, sondern auch zusammen mit den elastischen Haltemitteln als Abdichtung um die Hüfte.

**Patentansprüche**

1. Wegwerfwindel mit einer flüssigkeitsdurchlässigen oberen Lage (1), einer flüssigkeitsundurchlässigen unteren Lage (2), einem zwischen beiden Lagen eingelegten Saugkern (3) und mit im Schrittbereich an einander gegenüberliegenden Seiten angordneten, ein Auslaufen verhindernden Mitteln, die eine Tasche (14) ausbilden und jeweils eine biegsame Klappe (7) und ein oder mehrere elastische Teile (11) aufweisen, **gekennzeichnet** durch die folgenden Merkmalskombinationen:
   - die Klappe (7) weist einen sich von der Windel nach oben erstreckenden (abstehenden) Stützabschnitt (8), einen inneren, sich von dem Stützabschnitt (8) nach innen erstreckenden inneren Zweigabschnitt (9) und einen äußeren, sich von dem Stützabschnitt (8) nach außen erstreckenden Zweigabschnitt (10) auf;
   - das eine oder die mehreren elastische(n) Teil(e) (11) ist(sind) zumindest teilweise in den inneren und äußeren Zweigabschnitten eingearbeitet und bilden elastische Falten; und
   - zumindest die jeweiligen Innenflächen des Stützabschnitts (8) und des inneren Zweigabschnitts (9) bilden die Tasche (14) zur Aufnahme von Ausscheidungen.

2. Windel nach Anspruch 1, dadurch **gekennzeichnet,** daß das oder die elastische(n) Teil(e) entlang der seitlichen Randkanten der inneren und der äußeren Zweigabschnitte (9,10) längs der Mittenbereiche angeordnet ist(sind).

3. Windel nach Anspruch 1, dadurch **gekennzeichnet,** daß jeder Stützabschnitt (8) mit einem zugeordneten Basisabschnitt (13) verbunden ist, der sich von der zugeordneten Außenrandkante des Saugkerns (3) nach außen erstreckt.

4. Windel nach Anspruch 3, dadurch **gekennzeichnet,** daß der Basisabschnitt (13) zumindest teilweise von der unteren Lage (2) gebildet ist.

5. Windel nach mindestens einem der Ansprüche 1 bis 4, dadurch **gekennzeichnet,** daß die Klappe (7), die jede der ein Auslaufen verhindernden Mittel bildet, zumindest teilweise luftdurchlässig und wasserundurchlässig ausgebildet ist.

6. Windel nach mindestens einem der Ansprüche 1 bis 5, dadurch **gekennzeichnet,** daß jeder der inneren und äußeren Zweigabschnitte (9,10) an zumindest einem Längsende an einem Hauptkörper der Windel befestigt ist.

7. Windel nach mindestens einem der Ansprüche 1 bis 6, dadurch **gekennzeichnet,** daß der innere und der äußere Zweigabschnitt (9,10) von zumindest einer Hülse oder einem von einer Lage gebildeten Umschlag geformt ist und daß das (die) elastische(n) Teil(e) in der Hülse oder dem Umschlag eingearbeitet ist(sind).

8. Windel nach mindestens einem der Ansprüche 1 bis 7, dadurch **gekennzeichnet,** daß die ein Auslaufen verhindernden Mittel ebenfalls entlang der Taillen- oder Hüftlinie der Windel vorgesehen sind.

## Claims

1. A disposable nappy with an upper layer (1) permeable to liquid, a lower layer (2) impermeable to liquid, an absorbent core (3) disposed between the two layers and with means arranged on opposite sides in the crotch region to prevent leakage, which form a pocket (14) and each comprise a flexible flap (7) and one or more elastic parts (11), characterized by the following combination of features:
   - the flap (7) comprises a support section (8) extending up (standing out) from the nappy, an inner wing section (9) extending inwardly from the support section (8) and an outer wing section (10) extending outwardly from the support section (8);
   - the one or more elastic parts (11) is or are at least partially incorporated in the inner and outer wing sections and form elastic folds; and
   - at least the respective inner surfaces of the support section (8) and the inner wing section (9) form the pocket (14) for receiving excreta.

2. A nappy according to claim 1, characterized in that the elastic part or parts is or are arranged along the lateral edges of the inner and the outer wing sections (9, 10), along the central region.

3. A nappy according to claim 1, characterized in that each support section (8) is connected to an associated base section (13) which extends outwardly from the associated outer edge of the absorbent core (3).

4. A nappy according to claim 3, characterized in that the base section (13) is formed at least partially from the lower layer (2).

5. A nappy according to at least one of claims 1 to 4, characterized in that the flap (7) which forms each of the means preventing leakage is formed at least partially permeable to air and impermeable to water.

6. A nappy according to at least one of claims 1 to 5, characterized in that each of the inner and outer wing sections (9, 10) is fixed at at least one longitudinal end to a main body of the nappy.

7. A nappy according to at least one of claims 1 to 6, characterized in that the inner and the outer wing sections (9, 10) are formed from at least one sheath or from an envelope formed from one layer and in that the elastic part(s) is or are incorporated in the sheath or envelope.

8. A nappy according to at least one of claims 1 to 7, characterized in that the means preventing leakage are also provided along the waist or hip line of the nappy.

## Revendications

1. Couche-culotte jetable comportant une couche supérieure (1) perméable aux liquides, une couche inférieure (2) imperméable aux liquides, un corps absorbant (3) inséré entre ces deux couches, et des moyens disposés sur les côtés mutuellement opposés, dans la région de l'entrejambe, et empêchant les fuites, qui forment une poche (14) et présentent, respectivement, un rabat souple (7) et un ou plusieurs éléments élastiques (11), caractérisée par la combinaison des caractéristiques suivantes :
   - le rabat (7) comprend une section ou portion de soutien (8) qui s'étend en direction du haut en s'écartant de la couche-culotte, une section ou portion latérale intérieure (9) qui s'écarte de la section ou portion de soutien (8) en direction de l'intérieur, et une section ou portion latérale extérieure (10) qui s'écarte de la portion de soutien (8) en direction de l'extérieur ;
   - l'élément ou les éléments élastique(s) (11) est (sont) placé(s) au moins partiellement dans les sections ou portions latérales intérieure et extérieure et forment des fronces élastiques ; et
   - au moins les faces intérieures respectives de la section ou portion de soutien (8) et de la section ou portion latérale intérieure (9) forment la poche (14) pour recevoir les excrétions.

2. Couche-culotte suivant la revendication 1, caractérisée en ce que l'élément ou les éléments élastique(s) est (sont) disposé(s) le long des bords latéraux des sections ou portions latérales (9, 10) intérieure et extérieure, dans les régions centrales.

3. Couche-culotte suivant la revendication 1, caractérisée en ce que chaque section ou portion de soutien (8) est rattachée à une section ou portion de base (13) qui lui est associée, qui s'écarte du bord extérieur correspondant du corps absorbant (3), en direction de l'extérieur.

4. Couche-culotte suivant la revendication 3, caractérisée en ce que la section ou portion de base (13) est formée, au moins partiellement par la couche inférieure (2).

5. Couche-culotte suivant au moins l'une des revendications 1 à 4, caractérisée en ce que le rabat (7) qui forme chacun des moyens antifuites est, au moins partiellement perméable à l'air et imperméable à l'eau.

6. Couche-culotte suivant au moins l'une des revendications 1 à 5, caractérisée en ce que chacune des sections ou portion latérales (9, 10) intérieure et extérieure est fixée, au moins sur une extrémité longitudinale, à un corps principal de la couche-culotte.

7. Couche-culotte suivant au moins l'une des revendications 1 à 6, caractérisée en ce que les sections ou portions latérales (9, 10) intérieure et extérieure sont constituées par au moins un fourreau ou un ourlet formé par l'une des couches, et en ce que l'élément ou les éléments élastique(s) est (sont) placés dans ledit fourreau ou dans ledit ourlet.

8. Couche-culotte suivant au moins l'une des revendications 1 à 7, caractérisée en ce que des moyens antifuites sont également prévus sur la ligne de la taille ou des hanches de la couche-culotte.

# FIG.1

# FIG.2

# FIG.3

# FIG.4

# FIG.11

# FIG.12

# FIG.5

# FIG.6

# FIG.9

# FIG.7

# FIG.10

# FIG.8